# EUROPEAN PATENT APPLICATION

(11) **EP 4 606 902 A1**
(43) Date of publication of application: **27.08.2025**
(21) Application number: 24158908.4
(22) Date of filing: 21.02.2024
(51) Int. Cl.: C12P 17/02, C12P 17/10, C12P 17/12

(54) **PREPARATION OF ENANTIO-SPECIFIC EPOXIDES**

(71) Applicant: Vilnius University, 01513 Vilnius (LT)
(72) Inventor: Petkevicius, Vytautas, Vilnius (LT); Meskys, Rolandas, Vilnius (LT); Maciuityte, Greta, Vilnius (LT)
(74) Representative: AAA Law

(57) **Abstract**

A method for converting non-conjugated alkenes into enantio-specific epoxides by the use of monooxygenases.

## Description

### FIELD OF THE INVENTION

This invention relates to the field of biochemistry, more specifically to methods for converting alkenes into epoxides. More particularly, the present invention relates to converting non-conjugated alkenes into enantio-specific epoxides by the use of monooxygenase.

### BACKGROUND OF THE INVENTION

### Epoxides

Epoxides, compounds featuring a reactive three-membered oxygen ring, are highly sought-after compounds that play a versatile role as intermediates for a number of industrial chemical syntheses, including the production of pharmaceuticals, agrochemicals, and polymers. A classic method for synthesizing racemic epoxides is to expose alkenes to peroxyacids, such as peroxybenzoic acid (Wade, Organic Chemistry, 504-506, Prentice-Hall, Inc. (1990)).

A versatility of epoxides as synthons to obtain bioactive molecules and natural products is due to the presence of the chiral center, these compounds enable the synthesis of valuable enantiomers through ring-opening or functional group transformation reactions. This control over stereochemistry is pivotal in the production of enantiopure compounds, diminishing the potential for side effects and enhancing therapeutic outcomes. For example, the efficacy and safety of β-blockers, drugs used in cardiovascular conditions, depend on the specific isomer produced, with the S enantiomer typically exhibiting significantly higher potency (10-500-fold) than the R enantiomer. A crucial aspect of β-blocker production involves the stereospecific synthesis of a precursor - the appropriate epoxide. The precursors for β-blockers share a common structural feature: an unconjugated/unfunctionalized alkene moiety attached to the aryl scaffold usually via ether bond. Actually, the direct (stereo)selective epoxidation of unconjugated/unfunctionalized terminal alkenes using conventional chemical methods poses challenges due to their low reactivity towards electrophilic oxidation and the difficult chiral face control. Additionally, traditional epoxidation reactions involve hazardous materials and generate toxic waste. Conversely, biocatalysis represents a rapidly expanding field in sustainable technology. A notable distinction is the (stereo)selective synthesis of epoxides using oxygenases, commonly referred to as epoxidases. Yet, numerous prior studies and reports have emphasized the challenges of known epoxidases such as styrene monooxygenases, cytochrome P450s, and unspecific peroxygenases in converting nonactivated aryl alkenes into appropriate epoxides, despite the demand for enzymes with these capabilities. Overall, the range of reported epoxidases is limited, focused on a small set of starting materials such as styrene derivatives, terpenes, and aliphatic alkenes. This restricted availability of enzymes poses a significant challenge in selecting an appropriate biocatalyst with the desired selectivity, stability, solubility, and compatibility with specific expression hosts. Thus, the extensive application of epoxidases in biocatalytic synthesis has not been fully realized. Due to the scarcity of scientific research, particularly in the field of enzymes capable of epoxidizing unconjugated/unfunctionalized alkene moieties, the progress and adoption of environmentally friendly epoxidation methods for pharmaceuticals and fine chemicals face obstacles.

### Epoxidation by means of heme proteins

Despite numerous enzymes carry a heme cofactor, the most prominent and best-studied heme proteins with biotechnological relevance are the cytochrome P450 (CYP) enzymes. The heme-thiolato unit entails a unique reactivity, relying on the redox versatility of the heme-bound iron atom. Albeit countless studies have contributed to elucidating the catalytic cycle of CYPs in general and on the manifold reactions that CYPs can catalyze in specific, it is not fully understood, and many details are still under debate. Nevertheless, a few aspects of this highly complex redox cycle are accepted to contribute to their ability to perform epoxidation reactions. Epoxidation events frequently coincide with a degree of hydroxylation activity (which may be considered the "classical" monoogygenase activity of P450's), and the preference for one or the other reactivity is subject of many mechanistic studies [REFS here in sum], as are efforts to enhance their reactive selectivity. Since heme enzymes are a very prominent research subject, manyfold recent reviews are available to the interested reader and we refer to those wherever possible for more detailed insight.

Within the catalytic cycle of CYPs, the contribution of central intermediates are discussed. CYPs are able to utilize molecular oxygen (monooxygenase activity) as well as hydrogen peroxide (peroxygenase activity) or even higher peroxy compounds to transfer one oxygen onto their substrate. Irrespective of the oxygen source, coordination of the oxygen species results in "compound 0", a Felll(OOH) hydroperoxide. Upon cleavage of the O-OH bond, water is released and the heme-oxygen unit should classically be converted into "compound I", [FeIV(O)]·+, as oxidant to the substrate. Especially for epoxidation events, the involvement of an intermediate, the FeIII(H2O2) transient intermediate, has been assigned to play a crucial role as it provides a branch point towards formation of (productive) "compound I" as well as to (unproductive) uncoupling events. Substrate-olefin units in the active site are then subjected to nucleophilic attack. Another transient intermediate, an FeIV-alkoxy-radical complex, as alternative to "compound II", is discussed to result in epoxide release and restoration of the resting state of the enzyme. The orientation of the substrate within the active site is crucial, as the space it leaves available for the oxygen species contributes to the mode of oxygen binding and thus co-determines the nature of the oxygenation reaction, the regio- and the stereoselectivity of the newly formed substrate-oxygen bond(s).

### Monooxygenases P450cam: (https://pubs.acs.org/doi/10.1021/acs.jpcb.8b04279)

Many human CYP enzymes have been identified that promote the epoxidation of fatty acids. From a medicinal point of view, these reactions are discussed to be involved in e.g. cardiovascular diseases, neurodegeneration and inflammation processes. For example, two representatives of the human CYP4F subfamily were identified that exert epoxidation activity towards several (poly)unsaturated FAs. Upon heterologous production in yeast, site-directed mutagenesis enabled the study of site selectivity. Bacterial CYPs, such as from Streptomyces avermitilis, have also demonstrated FA epoxidation activity.

### Epoxidation by means of non-heme metalloproteins

Non-heme metalloproteins are Nature's solution for activating dioxygen by employing transition metals, where enzymes with copper or iron active sites are most commonly found (R. H. Holm, P. Kennepohl, E. I. Solomon. Chem. Rev., 1996, 96, 2239). None-heme iron enzymes encompass the pivotal part of metalloproteins and they can be divided into mononuclear and dinuclear iron enzymes. Both groups catalyze remarkable scope of oxidative transformations that seem to be even broader than that associated with oxidative heme or flavoproteins (Bruijnincx PC, van Koten G, Gebbink RJ. Chem. Soc. Rev. 2008;37(12):2716-44.). From these two groups, the mononuclear non-heme iron oxygenases have received the most attention since the structural data and mechanistic insights of many different enzymes are available (Lundberg M, Borowski T. Coordination chemistry reviews. 2013 Jan 1;257(1):277-89, Buongiorno D, Straganz GD. Coordination chemistry reviews. 2013 Jan 15;257(2):541-63). Majority of the enzymes utilize a common facial triad to achieve the activation of dioxygen (K. D. Koehntop, J. P. Emerson and L. Que, Jr, J. Biol. Inorg. Chem., 2005, 10, 87). This motif also known as the '2-His-1-carboxylate facial triad' consists of a mononuclear iron(II) metal center that is coordinated by two histidine residues and one carboxylate (glutamate or aspartate) residue. The mononuclear non-heme iron enzymes are divided into two classes on the basis of whether they utilize organic cofactor (either α-ketoglutarate or tetrahydrobiopterin pterin) or do not use organic cofactors (Rieske dioxygenases, extradiol dioxygenases). Several Fe2+-α-ketoglutarate-dependent oxygenases, involved in the production of natural compounds, have been linked to epoxide formation. AsqJ, a well-characterized enzyme from Aspergillus nidulans, catalyzes sequential desaturation and epoxidation of 4'-methoxycyclopeptin to yield (-)-4'-methoxycyclopenin which later undergoes several rearrangements to form 4'-methoxyviridicatin (Ishikawa N, Tanaka H, Koyama F, Noguchi H, Wang CC, Hotta K, Watanabe K. Angewandte Chemie International Edition. 2014 Nov 17;53(47):12880-4), containing a pharmaceutically attractive scaffold found in a variety of quinolone alkaloids (An CY, Li XM, Luo H, Li CS, Wang MH, Xu GM, Wang BG. Journal of Natural Products. 2013 Oct 25;76(10):1896-901). Mechanistic and computational studies of AsqJ have confirmed the key role of the ferryl (Fe(IV)-oxo species) intermediate in both enzymatic (desaturation and epoxidation) reactions. The proposed FeIV=O species completes the first hydrogen atom transfer (HAT) and forms Fe(III)-OH complex. The following catalytic preference of desaturation over hydroxylation seems to rely on the decay pathway after the initial C-H activation step. A similar desaturation-epoxidation step is found in the biosynthesis of sesquiterpenoid antibiotic pentalenolactone, catalyzed by the Fe2+-α-ketoglutarate-dependent dioxygenases PenD, PntD, and PtID.8 The α-ketoglutarate-dependent epoxidation was also described in the biosynthesis of natural antibiotic Nβ-epoxysuccinamoyl-DAP-Val (DAP - 2,3-diamino propionate) (Hollenhorst MA, Bumpus SB, Matthews ML, Bollinger Jr JM, Kelleher NL, Walsh CT. Journal of the American Chemical Society. 2010 Nov 10;132(44):15773-81). Authors have confirmed that α-KG-dependent enzyme DadC acts as an epoxidase and transforms the covalently bound Nβ-fumaramoyl-L-2,3-diaminopropionyl-S-DdaD species to generate Nβ-epoxysuccinamoyl-DAP. In this case, desaturation was unnecessary as the substrate already possesses a C=C double bond. The final stage in the biosynthesis of fosfomycin ((1R,2S)-epoxypropylphosphonate) in Streptomyces wedmorensis is catalyzed by HPP epoxidase.10 HPP is non-heme oxygenase, possessing a classical 2-His-1-carboxylate facial triad, though it catalyzes an unusual 1,3-dehydrogenation of the secondary alcohol in the (S)-2-hydroxypropyl-1-phosphonate to form epoxide ring. Authors also have shown that the oxidizing co-substrate for HppE is actually a hydrogen peroxide instead of oxygen, which was speculated to enable the bypass of the first two oxidation states of non-heme iron center and proceed directly to the ferryl complex (Wang C, Chang WC, Guo Y, Huang H, Peck SC, Pandelia ME, Lin GM, Liu HW, Krebs C, Bollinger Jr JM. Science. 2013 Nov 22;342(6161):991-5). Epoxidation of the benzene ring in aromatic compounds can also be attributed to some of the mononuclear iron enzymes, however, the resulting arene oxides are highly reactive and usually serve only as intermediates (Liu G, Zhao YL, He F, Zhang P, Ouyang X, Tang H, Xu P. Nature Communications. 2021 Feb 26;12(1):1301, Bassan A, Blomberg MR, Siegbahn PE. Chemistry-A European Journal. 2003 Sep 5;9(17):4055-67). Noteworthy, the targeted biotransformation of such reaction was achieved with biphenyl dioxygenase (BphA1A2A3A4) from P. pseudoalcaligenes KF707 as it showed unusual monooxygenase activity (Han J, Kim SY, Jung J, Lim Y, Ahn JH, Kim SI, Hur HG. Applied and Environmental Microbiology. 2005 Sep;71(9):5354-61) Normally, mononuclear iron(II) dioxygenases produce cis-dihydrodiols from aromatic substrates such as toluene, naphthalene, biphenyl, and flavones (Boyd DR, Bugg TD. Organic & biomolecular chemistry. 2006;4(2):181-92). The biotransformation of flavanone derivatives (a structural analog of flavone) resulted in the formation of stable epoxide products, with an epoxide moiety between C2' and C3' on the B ring (Han J, Kim SY, Jung J, Lim Y, Ahn JH, Kim SI, Hur HG. Applied and Environmental Microbiology. 2005 Sep;71(9):5354-61). Although mononuclear non-heme iron enzymes are rarely reported as epoxidation biocatalysts, their produced epoxide products are truly exceptional. In some aspects, these enzymes may be better biocatalysts for biotechnological applications than cytochrome P450 enzymes or FPMOs, due to the presence of a flexible iron center structure and the utilization of an abundant small metabolite (αKG, pterin) and the independence of redox partners in catalysis.

Non-heme dinuclear iron enzymes, unlike their mononuclear counterparts, are more enigmatic in terms of the availability of structural, biochemical, and mechanistic data, though they are more known for their epoxidation capabilities. Based on available genetic and structural information acquired to date, it appears that these enzymes consist of three or four components: a heterodimeric hydroxylase complex arranged in an (αβγ)2 or (αβ)2 quaternary structure, an NADH-oxidoreductase (in some cases with an N-terminal ferredoxin domain and/or a C-terminal reductase domain with FAD and NAD(P)-ribose binding regions), a small effector or coupling protein, and occasionally, a Rieske-type ferredoxin protein (Leahy JG, Batchelor PJ, Morcomb SM. FEMS microbiology reviews. 2003 Oct 1;27(4):449-79. Nichol T, Murrell JC, Smith TJ. European journal of inorganic chemistry. 2015 Jul;2015(21):3419-31. Osborne CD, Haritos VS. Molecular phylogenetics and evolution. 2019 Oct 1;139:106527). The active site, the carboxylate-bridged dinuclear iron center, is located deep within a four-helix bundle in the α subunit. Different groups In order to initiate the assimilation of diverse compounds as carbon sources, monooxygenases from distinct groups diiron enzymes harness various forms of iron-oxygen species resulting in different oxygenation reactions (Leahy JG, Batchelor PJ, Morcomb SM. FEMS microbiology reviews. 2003 Oct 1;27(4):449-79.) To achieve a challenging C-H bond breakage in methane, soluble methane monooxygenases (sMMO) generate a FeIV "diamond core" structure called compound Q, which is considered to be the most powerful oxidant found in nature (Rosenzweig AC. Nature. 2015 Feb 19;518(7539):309-10) Nevertheless, it was also demonstrated that sMMO enzymes can utilize the high-spin iron(iii) peroxo-bridged intermediate, designated as Hperoxo. Based on numerous experimental shreds of evidence it was suggested that Hperoxo is a more electrophilic oxidant than Q, preferring to react by a two-electron pathway (e. g. producing epoxides from alkenes), whereas one-electron oxidation (e.g. conversion of methane to methanol) processes are preferred by Q. Hence, in addition to natural hydroxylation of alkanes, sMMO can be exploited to transform of short-chain alkenes to produce epoxides. MMOs of Methylococcus capsulatus (Bath) and Methylosinus trichosporium OB3b were able to convert ethene, propene, 1-butene, and halogenated allyls to appropriate epoxides. When 1,3-butadiene was used, no di-epoxide was formed, suggesting that 1,2-epoxybut-3-ene could not be the substrate. 1-Pentene, 3-methyl-1-butene, and cyclohexene were not oxidized by these sMMO enzymes as well. Also, a similar set of alkenes was transformed to relevant epoxides by butane monooxygenase (BMO) of Pseudomonas butanovora and alkane monooxygenase (AlkB) of Pseudomonas oleovorans GPo. Although diiron(IV) species have not yet been experimentally confirmed, based on phylogenetic analysis and reaction products, propane/alkane and butane monooxygenases also utilize both Q-type and Hperoxo intermediates. However, this ability to harness compound Q and Hperoxo may render the epoxidation potential due to selectivity issues since in all of the aforementioned cases some of the desired epoxides were accompanied/dominated by the terminal alcohol products arising from the C-H oxidation. The inability of enzymes from other subgroups of diiron monooxygenases to convert methane suggests that Q intermediate does not occur in these systems and Hperoxo-type species are harnessed instead, leading to selective epoxidation if a substrate with C=C bonds is present. Several toluene monooxygenases (ToMOs) have been shown to form exclusively epoxides from ethene, propene, 1-butene, 2-butene, and 1,3-butadiene. Additionally, unlike sMMO enzymes, ToMOs converted bulkier substrates (e. g. 1-pentene, 2-pentene, and 1-hexene), but 1,7-octadiene was not oxidized. However, both 1,7-octadiene and 1-octene were substrates for AlkB. Interestingly enough, the aromatic compounds fell into the scope of ToMOs as well as sMMO enzymes and their more closely related counterparts. Production of styrene oxide was traced to the activity toluene of 4-monooxygenase (T4MO) from Pseudomonas mendocina KR1, propene monooxygenase (PMO) from Mycobacterium sp. strain M156, alkene monooxygenase XAMO from Xanthobacter autotrophicus Py2, but also MMO of Methylococcus capsulatus (Bath) and alkane monooxygenase AlkB. Furthermore, AlkB was able to epoxidize allylbenzene, benzyl allyl ether, and phenyl allyl ether. Most recent epoxidation examples catalyzed by non-heme dinuclear iron enzymes include isoprene monooxygenase (IsoMO) from Rhodococcus sp. Strain AD45, phenol monooxygenase-type PmlABCDEF enzyme, and several phenylacetyl-CoA monooxygenases.

The present invention is related to the epoxidation of alkenes by enzymes which are effective in producing epoxides with high levels of enantiomeric specificity. The closest analogue to the invention is described in US7169591B1 where a method for converting alkenes into epoxides and, particularly, to convert alkenes into enantio-specific epoxides by the use of enzymes which may be in their naturally-occuring (native) form or in mutated form, such as a native or mutated non-haem diiron-containing monooxygenase is proposed. However aforementioned method is not applicable for bulky non-conjugated alkenes.

### SUMMARY OF THE INVENTION

This method is designed for preparation of a single enantiomeric species of an epoxide comprising contacting an alkene with a monooxygenase consisting of six subunits with amino acid sequences from the group SEQ ID NO:1-6, SEQ ID NO:7-12, and SEQ ID NO:13-18.

In one aspect, this invention provides a method for preparing an enantiomeric epoxide comprising contacting a non-conjugated alkene with an enzyme comprising a monooxygenase and recovering the epoxides produced. In preferred form, the monooxygenase is a monooxygenase consisting of six different subunits with amino acid sequences SEQ ID NO:1-6

Another aspect of this invention provides a method for preparing an enantiomeric epoxide comprising contacting a non-conjugated alkene with an enzyme comprising a monooxygenase and recovering the epoxides produced. In preferred form, the monooxygenase is a monooxygenase consisting of six different subunits with amino acid sequences SEQ ID NO:7-12.

Another aspect of this invention provides a method for preparing an enantiomeric epoxide comprising contacting a non-conjugated alkene with an enzyme comprising a monooxygenase and recovering the epoxides produced. In preferred form, the monooxygenase is a monooxygenase consisting of six different subunits with amino acid sequences SEQ ID NO:13-18.

Another aspect of this invention provides a process for preparing an enantiomeric epoxide comprising contacting a non-conjugated alkene of the following general formula: 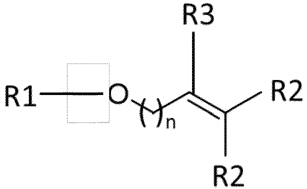 wherein R1 is C1-C12 alkyl, C1-C12 substituted alkyl, phenyl, (CH2)m-Ph, or substituted (CH2)m-Ph; wherein in said (CH2)m-Ph, m = 0, 1, 2, 3 to 10, Ph is phenyl; C1-C12 carboxyl, benzoyl or substituted benzoyl, pyridyl or substituted pyridyl, pyrimidyl or substituted pyrimidyl, thiophenyl or substituted thiophenyl, R2 is H, CH3; when R2 is H, R3 is H or CH3; n - 1-12; with an enzyme comprising a monooxygenase and recovering the epoxides produced.

Yet another aspect of this invention provides a process for preparing an enantiomeric epoxide comprising contacting a non-conjugated alkene of the following general formula: 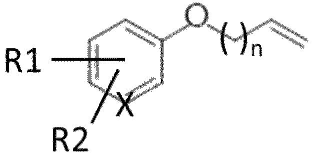 wherein R1 and R2 is H, F, Cl, Br, I, C1-C10 alkyl, C1-C10 substituted alkyl, -(CH2)m-Ph, or substituted -(CH2)m-Ph; wherein in said -(CH2)m-Ph, m = 0, 1, 2, 3 to 10, Ph is phenyl; alkoxy group with C1-C10 alkyl or C1-C10 substituted alkyl; -SCH3, -CCH3, -CN, cyclopropyl, cyclobutyl, cyclopentyl, cyclohexyl;
substituents R1 and R2 can be identical or different; n - 1-12; X - C or N; with an enzyme comprising a monooxygenase and recovering the epoxides produced.

One yet another aspect of this invention provides a process for preparing an enantiomeric epoxide comprising contacting a non-conjugated alkene of the following general formula: 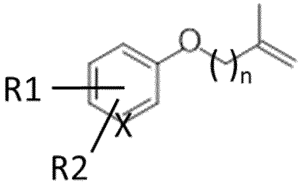 wherein R1 and R2 is H, F, Cl, Br, I, C1-C10 alkyl, C1-C10 substituted alkyl, -(CH2)m-Ph, or substituted -(CH2)m-Ph; wherein in said -(CH2)m-Ph, m = 0, 1, 2, 3 to 10, Ph is phenyl; alkoxy group with C1-C10 alkyl or C1-C10 substituted alkyl; -SCH3, -CCH3, -CN, cyclopropyl, cyclobutyl, cyclopentyl, cyclohexyl;
substituents R1 and R2 can be identical or different; n - 1-12; X - C or N; with an enzyme comprising a monooxygenase and recovering the epoxides produced.

Another aspect of this invention provides a process for preparing an enantiomeric epoxide comprising contacting a non-conjugated alkene of the following general formula: 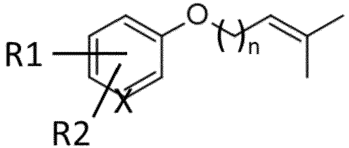 wherein R1 and R2 is H, F, Cl, Br, I, C1-C10 alkyl, C1-C10 substituted alkyl, -(CH2)m-Ph, or substituted -(CH2)m-Ph; wherein in said -(CH2)m-Ph, m = 0, 1, 2, 3 to 10, Ph is phenyl; alkoxy group with C1-C10 alkyl or C1-C10 substituted alkyl; -SCH3, -CCH3, -CN, cyclopropyl, cyclobutyl, cyclopentyl, cyclohexyl;
substituents R1 and R2 can be identical or different; n - 1-12; X - C or N; with an enzyme comprising a monooxygenase and recovering the epoxides produced.

Another aspect of this invention provides a process for preparing an enantiomeric epoxide comprising contacting a non-conjugated alkene of the following general formula: 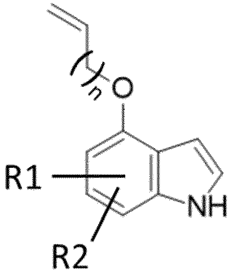 wherein R1 and R2 is H, F, Cl, Br, I, C1-C10 alkyl, C1-C10 substituted alkyl, -(CH2)m-Ph, or substituted -(CH2)m-Ph; wherein in said -(CH2)m-Ph, m = 0, 1, 2, 3 to 10, Ph is phenyl; alkoxy group with C1-C10 alkyl or C1-C10 substituted alkyl; -SCH3, -CCH3, -CN, cyclopropyl, cyclobutyl, cyclopentyl, cyclohexyl;
substituents R1 and R2 can be identical or different; n - 1-12 with an enzyme comprising a monooxygenase and recovering the epoxides produced.

Yet another aspect of this invention provides a process for preparing an enantiomeric epoxide comprising contacting a non-conjugated alkene of the following general formula: 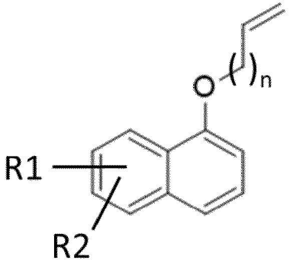 wherein R1 and R2 is H, F, Cl, Br, I, C1-C10 alkyl, C1-C10 substituted alkyl, -(CH2)m-Ph, or substituted -(CH2)m-Ph; wherein in said -(CH2)m-Ph, m = 0, 1, 2, 3 to 10, Ph is phenyl; alkoxy group with C1-C10 alkyl or C1-C10 substituted alkyl; -SCH3, -CCH3, -CN, cyclopropyl, cyclobutyl, cyclopentyl, cyclohexyl;
substituents R1 and R2 can be identical or different; n - 1-12 with an enzyme comprising a monooxygenase and recovering the epoxides produced.

Another aspect of this invention provides a process for producing a desired ratio of epoxide enantiomers comprising contacting a non-conjugated alkene with an enzyme comprising a monooxygenase and recovering the epoxides produced

In yet another aspect of this invention provides a process for producing a desired ratio of epoxide enantiomers (> 40% ee for (S)-enantiomer) comprising contacting a non-conjugated alkene with an enzyme comprising a monooxygenase and recovering the epoxides produced. In yet another aspect of this invention provides a process for producing a desired ratio of epoxide enantiomers (> 90% ee for (S)-enantiomer) comprising contacting a non-conjugated alkene with an enzyme comprising a monooxygenase and recovering the epoxides produced. Another aspect of this invention provides novel epoxides formed.

### DETAILED DESCRIPTION OF THE INVENTION

The present invention relates to monooxygenases, which convert alkenes into enantio-specific (S)-epoxides and methods using these enzymes. In particular, the present invention relates to monooxygenase enzymes, for example, PmlABCDEF (Pml), KatABCDEF (Kat) and MilABCDEF (Mil) monooxygenases, which may be used to convert alkenes into enantio-specific epoxides (S-epoxides), including the use of enzymes in a reaction with an alkene and recovery of the products of the reaction.

The ability to prepare the desired enantiomeric species of an epoxide provides for methods of large-scale production of desired epoxides which are useful in many processes, in particular, synthetic organic chemistry and pharmaceutical reactions.

### Enzymes Useful in the Practice of the Invention

The enzymes used in the present invention comprise native monooxygenases capable of oxidizing an alkene to an epoxide.

The term "monooxygenase" refers to an enzyme which catalyses the incorporation of one atom of oxygen into a molecule of substrate, the other oxygen being reduced to water.

There are a variety of methods known to those of skill in the art for determining whether an enzyme present in a microorganism can oxidize an alkene to an epoxide. These methods involve culturing a microorganism with a given alkene. Usually these methods involve introducing the grown-up microorganism into a sealed vessel, adding the alkene to the vessel followed by an incubation period. A gas or liquid sample is then taken from the vessel and analysed using a gas or liquid chromatograph which identifies the compounds present in the sample. One can then determine whether or not an enzyme present in the microorganism has transformed the alkene into a given epoxide species and the rate of conversion of the alkene into an epoxide can be measured. A chromatograph with a chiral separation column may be used to determine the enantiomeric ratios of the epoxides present in the sample.

Although a variety of monooxygenase enzymes are useful in the practice of the present invention, variants of PmlABCDEF (Pml), KatABCDEF (Kat) and MilABCDEF (Mil) monooxygenases, are particularly preferred in the practice of the present invention. The species of monooxygenase disclosed in the present application oxidize

The DNA sequences encoding PmlABCDEF (Pml) monooxygenase have been reported previously. The holozyme functions as an electron transport chain that shuttles electrons donated from NADH to the terminal sub-unit, which facilitates the specific epoxidation of alkenes through a reactive species of oxygen. The structure of the operon encoding the enzyme system, the enzyme amino acid sequence, and the basic catalytic mechanism of the enzyme are similar to several other diiron-containing enzymes including soluble methane monooxygenase (MMO) (Murrell, Biodegradation, 5:145-159 (1994); Lund et al., Eur. J. Biochem., 147:297-305 (1985); Zhou et al., FEBS Letters, 430:181-185 (1998)), alkene monooxygenases (Zhou et al., FEBS Letters, 430:181-185 (1998)), and toluene-2 and 3-monooxygenases (Shields et al., Appl. Environ. Microbiol., 55:1624-1629 (1996); Byrne et al., Gene, 154:65-70 (1995)). Knowledge of the enzyme structure provides a starting point for engineering the enzymes to alter their catalytic activity and possibly improve their enantioselectivity.

### Alkenes

A wide variety of alkenes may be utilized for epoxidation. For example, aromatic compounds or heterocycles harbouring 3, 4, 5, and 6 or longer carbon alkenes as radicals may be oxidized to their corresponding epoxides by PmlABCDEF (Pml), KatABCDEF (Kat) and MilABCDEF (Mil) monooxygenases. PmlABCDEF (Pml), KatABCDEF (Kat) and MilABCDEF (Mil) monooxygenases Table 2 presents the specific activity of a variety of PmlABCDEF (Pml), KatABCDEF (Kat) and MilABCDEF (Mil) monooxygenases against alkenes. To determine if a given alkene can be oxidized by a given monooxygenase, the procedure disclosed in Example 2 can be performed. Table3 presents data on the ratios of epoxide enantiomers produced in this type of reaction by PmlABCDEF (Pml), KatABCDEF (Kat) and MilABCDEF (Mil) monooxygenases. The epoxides formed in these reactions can undergo further oxidation reactions catalyzed by other enzymes resulting in the formation of enantio-pure diols which may be used in a variety of applications.

### Microorganisms

A variety of microorganisms can be modified to contain PmlABCDEF (Pml), KatABCDEF (Kat) and MilABCDEF (Mil) monooxygenases. In preferred embodiments, the microorganism is a bacterial species that naturally possesses a non-haem diiron monooxygenase or which is transformed with a DNA vector encoding PmlABCDEF (Pml), KatABCDEF (Kat) and MilABCDEF (Mil) monooxygenases.

Accordingly, microorganisms which have been transformed with a plasmid or other vector containing the gene(s) for PmlABCDEF (Pml), KatABCDEF (Kat) and MilABCDEF (Mil) monooxygenases may be used in the practice of the present invention. A procedure for transforming bacteria with PmlABCDEF (Pml), KatABCDEF (Kat) and MilABCDEF (Mil) monooxygenases genes is presented hereinbelow in Example 1. This procedure may be modified as necessary to introduce PmlABCDEF (Pml), KatABCDEF (Kat) and MilABCDEF (Mil) monooxygenases into a given bacterial species.

The PmlABCDEF (Pml), KatABCDEF (Kat) and MilABCDEF (Mil) monooxygenases is preferably maintained within a host microorganism that is contacted with the alkene(s). However, it is also possible to isolate the enzyme from the microorganism and use the isolated and purified enzyme, if desired.

### Large Scale Preparation of Epoxides

An exemplary use of the present invention is the production of epoxides in a bioreactor which includes microorganisms comprising PmlABCDEF (Pml), KatABCDEF (Kat) and MilABCDEF (Mil) monooxygenases to catalyze the conversion of alkenes to enantio-pure epoxides. By controlling the quantity of the alkene(s) introduced into the bioreactor and the contact time of the enzyme-containing microorganism with the alkene, a substantial yield of optically active epoxides may be obtained. Because the alkenes and the epoxides have vastly different boiling points, the parent and product compounds may be separated by distillation. In addition, other methods such as extraction using various solvents can be also applied. Thus, the present invention represents a method for using the powerful catalytic potential of the PmlABCDEF (Pml), KatABCDEF (Kat) and MilABCDEF (Mil) monooxygenases to generate useful products. A variety of methods for growing and maintaining microorganisms in a bioreactor are known to those of skill in the art.

Present invention defines a method for using monooxygenases, wherein the method comprises the steps:
a) the nucleic acid selection from the group encoding the SEQ ID NO:1-6, SEQ ID NO:7-12, or SEQ ID NO:13-18;
b) construction of a DNA vector harbouring the selected nucleic acid;
c) host cell transformation with the DNA vector from step (b);
d) preparation a single enantiomeric species of an epoxide by incubation of the host cell from step (c) with the alkene.

The following examples are given as illustrative of the present invention.

### EXAMPLES

### Example 1

### Determination of the Ability of PmlABCDEF (Pml), KatABCDEF (Kat), MilABCDEF (Mil) Monooxygenases to Oxidize Alkenes and Generate Epoxides

Table 1 below lists strains and plasmids useful in the practice of the invention.

**TABLE 1. Strains and Plasmids**

| Strains & Plasmids | Relevant Phenotype | Reference |
|---|---|---|
| pBAD2-MCS-1 | The amplicon containing regulatory elements of pBAD24 fused into the chassis of pBBR1MCS | Petkevičius et al. (2018) |
| Pml_pBAD2 | Recombinant pBAD2-MCS-1 containing *pmlABCDEF* gene | This work |
| Kat_pBAD2 | Recombinant pBAD2-MCS-1 containing *katABCDEF* gene | This work |
| Mil_pBAD2 | Recombinant pBAD2-MCS-1 containing *milABCDEF* gene | This work |
| *E. coli* DH5a | F⁻ *endA1 glnV44 thi-1 recA1 relA1 gyrA96 deoR nupG purB20* φ80d/*acZ*ΔM15 Δ(*lacZYA-argF*)U169, hsdR17(*r_{K}⁻m_{K}*⁺), λ⁻ | Thermo Fischer Scientific, Lithuania |
| *Pseudomonas putida* KT2440 | Plasmid-free derivative of a toluene-degrading bacterium strain *Pseudomonas putida* mt-2 | DSM 6125 |

### Preparation of Recombinant Bacteria Containing Monooxygenases

This example is illustrative of a procedure for preparing a recombinant microorganism that can oxidize alkenes to form epoxides.

In particular, this example presents a procedure used to introduce the monooxygenase genes and to induct conditions for the expression of PmlABCDEF (Pml), KatABCDEF (Kat), MilABCDEF (Mil) monooxygenase genes in Pseudomonas putida KT2440 strain.

This same procedure may be used to prepare other recombinant microorganisms containing the monooxygenase genes or similar genes that encode a monooxygenase capable of oxidizing an alkene to an epoxide.

Unless otherwise noted, all molecular biological manipulations were performed by methods known to those skilled in the art, essentially as described by Sambrook, et al. (Molecular Cloning, A Laboratory Manual, 2nd Edition, Cold Spring Harbor Laboratory Press, 1989.) The DNA sequences of pml ABCDEF have been reported previously (Petkevičius et al. 2019). The total genomic DNA from soil and sediment samples was isolated by using "ZR Soil Microbe DNA MidiPrep" (Zymo Research, Germany). Metagenomic libraries were constructed using a pUC19 vector as described previously (Urbeliene et al. 2019). The oxygenases producing hits forming blue colonies were screened by the spreading transformed *E*. *coli* cells on LB agar supplemented with indole (1 mM) and ampicillin (100 µg/ml). The plasmid DNA was isolated from each positive clone and the cloned fragment was partially sequenced from both ends. The six genes (pmlABCDEF, katABCDEF, or milABCDEF) were amplified by using polymerase chain reaction (PCR) with primers listed in Table2.

**TABLE 1. Prime sequences**

| Primer designation | Sequence 5' to 3' | Use |
|---|---|---|
| Pml_Xba_F | ATTATCTAGAAATAATTTTGTTTAACTTTAAGAAGGAGAT ATACCATGAGCGAGAAATCATTGG | Cloning |
| Pml_Hind_R | AATTAAGCTTTCAGATGGCCTTGAACAGC | Cloning |
| Kat_Xba_F | ATTATCTAGAAATAATTTTGTTTAACTTTAAGAAGGAGAT ATACCATG ACTGACACCGGCACCC | Cloning |
| Kat_Hind_R | AATTAAGCTTTCAGATGCGTTTGAACAGG | Cloning |
| Mil_Xba_F | ATTATCTAGAAATAATTTTGTTTAACTTTAAGAAGGAGAT ATACCATG AACTTGCCTGATGAGTCAC | Cloning |
| Mil_Hind_R | AATTAAGCTTTCAGACGGCTTTGAACAGGG | Cloning |

PCR was performed using a Phusion Green Hot Start II High-Fidelity PCR Master Mix (Thermo Fischer Scientific, Lithuania and reaction conditions recommended by the manufacturer. Cycling conditions were: initial denaturation 30 s at 98 °C., cycle steps 10 s at 98 °C., 10 s at 55 °C, and 2 min at 72 °C for 30 cycles, final elongation at 72 °C for 7 min. Amplified DNA was digested with Xbal and Hindlll
(Thermo Fischer Scientific, Lithuania), and ligated to similarly digested pBAD2-MCS-1. The ligation mixture was used to transform E. coli DH5α. Clones were selected by plating the cells onto LB agar-supplemented with kanamycin (40 µg/ml), and then replica plating onto LB plates that contained 100 µg/ml indole, and 0,2 % arabinose. A single colony that formed a blue color from the conversion of indole to indigo, indicating monooxygenase activity, and contained the 4,5 bp insert of PmlABCDEF (Pml), KatABCDEF (Kat), MiIABCDEF, as determined by restriction analysis and sequencing, was selected for further use and designated Pml_pBAD2, Kat_pBAD2, and Mil_pBAD2.

Pseudomonas putida KT2440 (DSM 6125, DSMZ, Germany) was used as a host for recombinant protein production and bioconversions. Arabinose-inducible plasmids Kat_pBAD2, Mil_pBAD2, and Pml_pBAD2, containing appropriate monooxygenase encoding genes (SEQ ID NO:1-6, SEQ ID NO:7-12, and SEQ ID NO:13-18, respectively) were introduced into the host via electroporation according to Choi et al. (2006) modified protocol. Then an overnight culture of appropriate Pseudomonas putida KT2440 transformant was placed into fresh LB medium (1/100 volume ratio) containing 40 µg mL-1 of kanamycin and 1% of glucose. The cultivation was executed for 3-4 h with shaking (180 rpm) at 30 °C until OD600 0.6-0.8 was reached and then L-arabinose was added to a final concentration of 0.2%. This was followed by the addition of FeSO4·7H2O to the final concertation of 0.1 mM and the bacterial culture was left to incubate overnight.

### Biotransformation conditions of whole-cell biocatalysis

After induction, cells were separated from the medium by centrifugation (4000 g for 10 min). The resulting biomass was washed twice with bioconversion buffer (glucose - 10,0 g L⁻¹, Na₂HPO₄- 3,6 g L⁻¹, KH₂PO₄ - 1,5 g L⁻¹, NH₄Cl - 1,0 g L⁻¹, pH = 7,5) and suspended in the same buffer. Parameters of the standard biotransformation procedure were as follows: cell concentration of 10 g L⁻¹_{CDW}, 20 mL of bioconversion buffer in glass flasks, incubation executed at 30 °C on a rotary shaker at 200 rpm for 16-24 h, final substrate concentration was 5-10 mM. Substrate stocks were prepared as 1 M solutions of the appropriate compound in ethanol.

### Alkene Oxidation Assays

To determine the range of alkenes that could be oxidized by the monooxygenase PmlABCDEF (Pml), KatABCDEF (Kat) and MilABCDEF (Mil) 300 -500 µL aliquots of the cultures were periodically removed from the shaker and analyzed with HPLC-MS and GC-MS. Verification of epoxide formation was carried out by the colorimetric assay using 4-(4-nitrobenzyl)pyridine (pNBP) (Alcalde et al. 2004). This allowed the monitoring of both the alkene compounds as well as the epoxide products.

### HPLC-MS analysis

The sample (0.5 mL) of the whole-cell biocatalysis reaction was transferred to a 1.5 mL tube and mixed with an equal part of acetonitrile. After the mixture was centrifuged (12000 g for 5 min), an injection of supernatant was analysed using a high-performance liquid chromatography system. HPLC-MS analysis was performed using a high-performance liquid chromatography system (Shimadzu, Japan) equipped with a photo diode array (PDA) detector and a mass spectrometer (LCMS-2020; Shimadzu) equipped with an ESI source. The data were analysed using the LabSolutions LCMS software.

### GC-MS analysis

Samples of the whole cell biocatalysis were extracted with an equal part of ethyl acetate or dichloromethane. After mixing they were vigorously shaken with a vortex-shaker (VWR, Germany) for 5 seconds. For phase separation, the mixture was spun down in a Micro Star 17 centrifuge (VWR, Germany) at 17000 *g,* 1 min. The solvent phase was taken up and transferred into a new reaction tube. The extraction solvent was further dried with a spatula tip of anhydrous MgSO₄, centrifuged again, and transferred into a 1.5 ml glass vial, topped with a septa cap.

GC-MS analysis was performed with a Shimadzu Nexis GC_2030 connected to a GCMS-QP2020 NX (Shimadzu, Japan) equipped with a FS-Supreme-5ms column (Ziemer, Germany). For gas chromatography, helium was used as a carrier gas with a linear velocity of 36 cm sec⁻¹.The injection volume was 1 µL with an injection port temperature of 350 °C and a split ratio of 1:50. For mass spectrometry, the MS was set to scan for m/z from 50 to 500 with an event tome of 0.3 seconds and a scan speed of 1666 scans per event. Solvent cut time was set for 2 min.

Table 3 presents the results of these assays indicating the activity of PmlABCDEF (Pml), KatABCDEF (Kat) and MilABCDEF (Mil) monooxygenases towards alkenes.

**TABLE 3. Activity of PmlABCDEF (Pml), KatABCDEF (Kat), MilABCDEF (Mil) Monooxygenases Towards Alkenes. Methods for product identification: A - detected by GC-MS, B - detected by HPLC-MS, C - detected by colorimetric assay using 4-(4-nitrobenzyl)pyridine (pNBP), D - determined by NMR.**

| Alkene | PmlABCDEF | KatABCDEF | MilABCDEF |
|---|---|---|---|
| 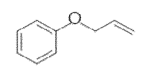 | B, C, D | B, C | B, C |
| 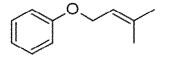 | A, B | B | B |
| 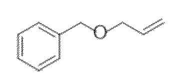 | B, C, D | B, C | B, C |
| 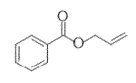 | A, B, C, D | B, C | B, C |
| 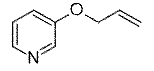 | B, C, D | B, C | B, C |
| 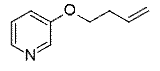 | B, C, D | B, C | B, C |
| 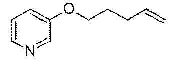 | B, C, D | B, C | B, C |
| 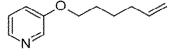 | B, C, D | B, C | B, C |
| 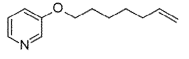 | B, C, D | B, C | B, C |
| 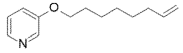 | B, C, D | B, C | B, C |
| 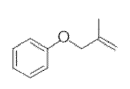 | A, B, C, D | B | B |
| 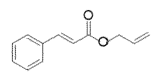 | B, C | B, C | B |
| 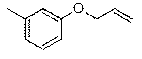 | A, B, C | A, B, C | A, B, C |
| 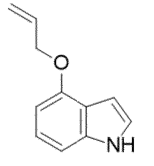 | B, C | B, C | B, C |
| 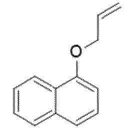 | B, C | B, C | B, C |

### Example 2

### Chemical synthesis of 2-(3-methylphenoxymethyl)oxirane and 3-(2-oxiranylmethoxy)pyridine racemic and enantiomerically enriched standards

Racemic and enantiomerically enriched 2-(3-methylphenoxymethyl)oxirane was synthesized from *m*-cresol and appropriate epichlorohydrins. To a solution of *m*-cresol (1.0 equiv.) in DMF was added sodium hydride (1.5 equiv., 60% in mineral oil) at 0 °C. The reaction mixture was stirred for 30 min and warmed to room temperature and epichlorohydrin (2.0 equiv.) was added dropwise over 2 minutes. After the starting material was consumed (as monitored by TLC), the mixture was poured into ice water (50 mL), and extracted with ethyl acetate (3 × 50 mL). The combined organic phase was dried over anhydrous sodium Na₂SO₄ and concentrated under a vacuum. The residue was purified by silica gel column chromatography with ethyl acetate/hexane (10:90) eluent to give the final product as colorless oil in 60-70 % yield. Enantiomerically enriched (S)-2-(3-methylphenoxymethyl)oxirane was synthesized using (R)-epichlorohydrin, while enantiomerically enriched (R)-2-(3-methylphenoxymethyl)oxirane was synthesize using (S)-epichlorohydrin.

Racemic and enantiomerically enriched 3-(2-oxiranylmethoxy)pyridine was synthesized from 3-pyridinol and appropriate epichlorohydrins. To a solution of 3-pyridinol (1.0 equiv.) in DMF was added sodium hydride (1.5 equiv., 60% in mineral oil) at 0 °C. The reaction mixture was stirred for 30 min and warmed to room temperature and epichlorohydrin (2.0 equiv.) was added dropwise over 2 minutes. After the starting material was consumed (as monitored by TLC), the mixture was poured into ice water (50 mL) and extracted with ethyl acetate (3 × 50 mL). The combined organic phase was dried over anhydrous sodium Na₂SO₄ and concentrated under a vacuum. The residue was purified by silica gel column chromatography with methanol/chloroform (10:90) eluent to give the final product as brown oil in 30-40 % yield. Enantiomerically enriched (S)-(2-oxiranylmethoxy)pyridine was synthesized using (R)-epichlorohydrin, while enantiomerically enriched (R)-(2-oxiranylmethoxy)pyridine was synthesize using (S)-epichlorohydrin.

HPLC analysis was performed using a high-performance liquid chromatography system (Shimadzu, Japan), and data were analyzed using the LabSolutions LCMS software. CHIRALCEL OD-H, 250 × 4.6 mm, 5 µm column (Daicel, Japan) was used for separation. An isocratic mixture of isopropanol/hexane (10:90) and a flow rate of 0.5 mL/min was used for 2-(3-methylphenoxymethyl)oxirane. The retention time for each enantiomer was as follows: t*_{R}* = 13.3 min, ts = 20.8 min. An isocratic mixture of isopropanol/hexane (5:95) and a flow rate of 0.5 mL/min was used for 3-(2-oxiranylmethoxy)pyridine separation. The retention time for each enantiomer was as follows: t*_{S}* = 54.2 min, t*_{R}* = 59.2 min.

### Chemical synthesis of 3-alkenyloxypyridines

Series of 3-alkenyloxypyridines (3-(allyloxy)pyridine, 3-but-3-enyloxy-pyridine 3-pent-4-enyloxy-pyridine, 3-hex-5-enyloxy-pyridine, 3-hept-6-enyloxy-pyridine, and 3-okt-7-enyloxy-pyridine) were synthesized form 3-pyridinol and appropriate alkenyl bromides. To a solution of 3-pyridinol (1.0 equiv.) in DMF was added sodium hydride (1.5 equiv., 60% in mineral oil) at 0 °C. The reaction mixture was stirred for 30 min and warmed to room temperature and appropriate alkenyl bromide (2.0 equiv.) was added dropwise over 10 minutes. After the starting material was consumed (as monitored by TLC), the mixture was poured into ice water (50 mL) and extracted with ethyl acetate (3 × 50 mL). The combined organic phase was dried over anhydrous sodium Na₂SO₄ and concentrated under a vacuum. The residue was purified by silica gel column chromatography with methanol/chloroform (10:90) eluent to give the final product. Product yield in a range of 30-50 % was observed for different 3-alkenyloxypyridines.

### Chemical synthesis of 4-(allyloxy)-indole

The targeted compound was obtained from a reaction between 4-hydroxyindole and allyl bromide. To a solution of 4-hydroxyindole (1.0 equiv.) in DMF was added sodium hydride (1.5 equiv., 60% in mineral oil) at 0 °C. The reaction mixture was stirred for 30 min and warmed to room temperature and allyl bromide (2.0 equiv.) was added dropwise over 10 minutes. After the starting material was consumed (as monitored by TLC), the mixture was poured into ice water (50 mL) and extracted with ethyl acetate (3 × 50 mL). The combined organic phase was dried over anhydrous sodium Na₂SO₄ and concentrated under a vacuum. The residue was purified by silica gel column chromatography with ethyl acetate/hexane (10:90) eluent to give the final product as yellow oil in 40-50 % yield. The purity was evaluated with HPLC-MS, and data matched those of the literature (Sun et al. 2020).

### Chemical synthesis of phenyl prenyl ether

The targeted compound was obtained from a reaction between phenol and 3,3-dimethylallyl bromide. To a solution of phenol (1.0 equiv.) in DMF was added potassium carbonate (1.5 equiv.) at room temperature. The reaction mixture was stirred for 30 min and 3,3-dimethylallyl bromide (2.0 equiv.) was added dropwise over 10 minutes. After the starting material was consumed (as monitored by TLC), the mixture was filtered, poured into ice water (50 mL), and extracted with ethyl acetate (3 × 50 mL). The combined organic phase was dried over anhydrous sodium Na₂SO₄ and concentrated under a vacuum. The residue was purified by silica gel column chromatography with ethyl acetate/hexane (10:90) eluent to give the final product as colorless oil in 70-80 % yield. The purity was evaluated with HPLC-MS, and data matched those of the literature (Klein et al. 2013, Klaper et al. 2016).

**3-(allyloxy)pyridine:** ¹H NMR (400 MHz, DMSO-d6): δ = 4.65 (dt, *J* = 5.3, 1.6 Hz, 2H), 5.29 (d, *J =* 10.5 Hz, 1H), 5.42 (d, *J =* 17.3 Hz, 1H), 6.05 (ddt, *J* = 17.3, 10.5, 5.3 Hz, 1H), 7.31 - 7.42 (m, 2H), 8.16 - 8.18 (m, 1H), 8.30 - 8.32 (m, 1H). ¹³C NMR (101 MHz, DMSO-d6): δ = 68.88, 118.41, 121.71, 124.57, 133.69, 138.46, 142.27, 154.91.

**3-(2-Oxiranylmethoxy)pyridine:** ¹H NMR (400 MHz, DMSO-d6): δ = 3.09 (3.09 (dd, J = 12.5, 7.0 Hz, 1H), 3.34 - 3.41 (m, 1H), 3.60 (dd, *J* = 12.4, 6.9 Hz, 1H)), 4.22 - 4.12 (m, 2H), 7.28 - 7.20 (m, 2H), 8.09 (d, *J* = 1.3 Hz, 1H), 8.32 (dd, *J* = 6.7, 2.3 Hz, 1H). ¹³C NMR (101 MHz, DMSO-d6): δ = 43.89, 50.45, 68.76, 121.82, 124.44, 137.61, 143.35, 153.55.

**3-But-3-enyloxy-pyridine:** ¹H NMR (DMSO-d6, 400 MHz): δ = 2.73 (s, *J =* 6.6 Hz, 2H), 4.10 (t, *J* = 6.6 Hz, 2H), 5.22 - 5.06 (m, 2H), 5.89 (ddt, *J* = 17.0, 10.3, 6.6 Hz, 1H), 7.31 (dd, *J =* 8.4, 4.6 Hz, 1H), 7.38 (ddd, *J* = 8.4, 3.0, 1.4 Hz, 1H), 8.16 (dd, *J* = 4.5, 1.4 Hz, 1H), 8.28 (d, *J* = 3.0 Hz, 1H). ¹³C NMR (DMSO-d6, 101 MHz): δ = 33.42, 67.46, 117.63, 121.42, 124.57,135.12, 138.30, 142.15, 155.16.

**3-(2-Oxiranylethoxy)pyridine:** ¹H NMR (DMSO-d6, 400 MHz): δ = 1.78 - 1.91 (m, 2H), 3.34 (dd, *J* = 12.3, 7.0 Hz, 1H), 3.58 (dd, *J =* 12.4, 7.0 Hz, 1H), 3.64 - 3.71 (m, 1H), 4,05 - 4,17 (m, 2H), 7.04 -7.40 (m, 2H), 8.10 (d, *J* = 1.7 Hz, 1H), 8.33 (dd, *J* = 7.0, 1.9 Hz, 1H). ¹³C NMR (DMSO-d6, 101 MHz): δ = 32.48, 46.94, 51.31, 65.97, 121.42, 124.23, 137.55, 143.50, 154.67.

**3-Pent-4-enyloxy-pyridine:** ¹H NMR (DMSO-d6, 400 MHz): δ = 1,82 (dd, *J* = 8.2, 6.5 Hz, 2H), 2.15 - 2.33 (m, 2H), 4.04 (t, *J* = 6.4 Hz, 2H), 4.97 - 5.02 (m, 1H), 5.06 (dd, *J* = 17.2, 1.8 Hz, 1H), 5.87 (ddt, *J* = 16.9, 10.2, 6.6 Hz, 1H), 7.31 (dd, *J =* 8.4, 4.5 Hz, 1H), 8.16 (d, *J* = 4.5 Hz, 1H), 8.32 (s, 2H). ¹³C NMR (DMSO-d6, 101 MHz): δ = 28.22, 29.98, 67.91, 115.76, 121.34, 124.58, 138.30, 138.32, 142.08, 155.29.

**3-(2-Oxiranylpropoxy)pyridine:** ¹H NMR (DMSO-d6, 400 MHz): δ = 1.51 -1.74 (m, 2H), 1.77 - 1.95 (m, 2H), 2.47 (dd, *J* = 5.1, 2.7 Hz), 2.70 (t, *J* = 4.5 Hz, 1H), 2.98 (qt, *J* = 4.5, 2.7 Hz), 4.08 (t, *J* = 6.4 Hz), 7.32 (dd, *J* = 8.4, 4.5 Hz, 1H), 7.38 (ddd, *J* = 8.5, 3.0, 1.5 Hz, 1H), 8.16 (dd, *J* = 4.5, 1.4 Hz, 1H), 8.29 (d, *J* = 3.0 Hz, 1H). ¹³C NMR (DMSO-d6, 101 MHz): δ =25.65, 28.95, 46.58, 51.68, 67.90, 121.39, 124.60, 138.29, 142.12, 155.23.

**3-Hex-5-enyloxy-pyridine:** ¹H NMR (DMSO-d6, 400 MHz): δ = 1.51 (p, *J =* 7.5 Hz, 2H), 1.73 (dt, *J* = 14.9, 6.6 Hz, 2H), 2,09 (q, *J* = 7.2 Hz, 2H), 4.04 (t, *J* = 6.5 Hz, 2H), 4.97 (dt, *J* = 10.3, 1.7 Hz, 1H), 5.04 (dq, *J* = 17.2, 1.8, 1H), 5.82 (ddt, *J* = 16.9, 10.2, 6.6 Hz, 1H), 7.31 (dd, *J =* 8.4, 4.5 Hz, 1H), 7.37 (ddd, *J* = 8.4, 2.9, 1.4 Hz, 1H), 8.15 (dd, *J* = 4.5, 1.4 Hz, 1H), 8.28 (d, *J* = 2.9 Hz, 1H). ¹³C NMR (DMSO-d6, 101 MHz): δ = 25.10, 28.49, 33.26, 68.06, 79.65, 115.43, 121.31, 124.56, 138.27, 138.96, 142.02.

**3-(2-Oxiranylbutoxy)pyridine:** ¹H NMR (DMSO-d6, 400 MHz): δ = 1.19-1.32 (m, 1H), 1.45 - 1.63 (m, 3H), 1.70 - 1.87 (m, 2H), 2.45 (dd, *J* = 5.1, 2.7 Hz, 1H), 2.68 (dd, *J* = 5.1, 4.0 Hz, 1H), 2.87 - 2.95 (m, 1H); 4.05 (t, *J* = 6.4 Hz, 2H), 7.32 (dd, *J* = 8.4, 4.5 Hz, 1H), 7.38 (ddd, *J* = 8.5, 3.0, 1.4 Hz, 1H), 8,16 (dd, *J* = 4.5, 1.4 Hz, 1H), 8.28 (d, *J =* 2.9, 1H). ¹³C NMR (DMSO-d6, 101 MHz): δ = 22.56, 28.80, 32.00, 46.52, 51.91, 68.11, 121.34, 124.52, 138.27, 142.04, 155.30.

**3-Hept-6-enyloxy-pyridine:** ¹H NMR (DMSO-d6, 400 MHz): δ = 1.32 - 1.49 (m, 4H), 1.73 (dd, *J* = 8.4, 5.2 Hz, 2H), 1.96 -2.10 (m, 2H), 4.03 (t, *J=* 6.5 Hz, 2H), 4.95 (dd, *J =* 10.2, 2.0 Hz, 1H), 5.02 (dq, *J* = 17.2, 1.8 Hz, 1H), 5.81 (ddt, *J* = 16.9, 10.1, 6.6 Hz, 1H), 7.31 (dd, J *=* 8.4, 4.5 Hz, 1H), 7.37 (ddd, *J =* 8.5, 3.0, 1.4 Hz, 1H), 8.14 - 8.17 (m, 1H), 8.28 (d, *J* = 2.9 Hz, 1H). ¹³C NMR (DMSO-d6, 101 MHz): δ = 25.42, 28.43, 28.82, 33.58, 68.20, 115.24, 121.31, 124.57, 138.27, 139.13, 142.01, 155.32.

**3-(2-Oxiranylpentoxy)pyridine:** ¹H NMR (DMSO-d6, 400 MHz): δ = 1.37 - 1.51 (m, 4H), 1.54 - 1.71 (m, 4H), 3.30 (dd, *J =* 12.3, 7.0 Hz, 1H), 3.49 (dd, *J* = 12.3, 7.0 Hz, 1H), 3.49 (dd, *J =* 12.3, 7.0 Hz, 1H), 4.04 (t, *J* = 7.0 Hz, 2H), 7.22 - 7.31 (m, 2H), 8.10 (d, *J=* 1.6 Hz, 1H), 8.33 (dd, *J* = 7.0, 2.0 Hz, 1H). ¹³C NMR (DMSO-d6, 101 MHz): δ = 25.38, 26.06, 29.38, 32.26, 47.09, 52.94, 68.24, 121.43, 124.39, 137.53, 142.74, 154.81.

**3-Oct-7-enyloxy-pyridine:** ¹H NMR (DMSO-d6, 400 MHz): δ = 1.28 - 1.46 (m, 6H), 1.67 - 1.76 (m, 2H), 2.02 (q, *J* = 7.0 Hz, 2H), 4.02 (t, *J* = 6.5 Hz, 2H), 4.90 - 4.97 (m, 1H), 5.00 (dt, *J* = 17.1, 1.9 Hz, 1H), 5.80 (ddt, *J* = 16.9, 10.2, 6.7 Hz, 1H), 7.31 (dd, *J* = 8.4, 4.5 Hz, 1H), 7.37 (ddd, *J* = 8.5, 3.0, 1.5 Hz, 1H), 8.15 (dd, *J* = 4.5, 1.4 Hz, 1H), 8.27 (d, *J* = 2.9 Hz, 1H). ¹³C NMR (DMSO-d6, 101 MHz): δ = 25.72, 28.68, 28.98, 33.58, 115.12, 121.30, 124.55, 138.27, 139.22, 142.00, 155.32.

**3-(2-Oxiranylhexoxy)pyridine:** ¹H NMR (DMSO-d6, 400 MHz): δ = 1.12- 1.26 (m, 1H), 1.34 - 1.54 (m, 7H), 1.72 (dt, *J* = 13.0, 6.4 Hz, 2H), 3.17 (s, 1H), 3.45 (q, *J=* 7.0 Hz, 1H), 3.57 (s, 1H), 4.03 (td, *J* = 6.8, 3.4 Hz, 2H), 7.28 - 7.41 (m, 2H), 8.15 (dd, *J* = 4.5, 1.5 Hz, 1H), 8.28 (d, *J =* 2.9 Hz, 1H). ¹³C NMR (DMSO-d6, 101 MHz): δ = 25.81, 25.92, 28.93, 28.98, 32.30, 46.56, 51.97, 68.21, 121.33, 124.37, 138.27, 142.01, 155.32.

**Phenyl glycidyl ether:** ¹H NMR (DMSO-d6, 400 MHz): δ = 2.71 (dd, *J* = 5.1, 2.7 Hz, 1H, CH), 2.84 (dd, *J* = 5.1, 4.2 Hz, 1H, CH), 3.24 - 3.42 (m, 1H, CH), 3.82 (dd, *J* = 11.3, 6.5 Hz, 1H, CH), 4.31 (dd, *J* = 11.3, 2.7 Hz, 1H, CH), 7.08 - 6.83 (m, 3H, CH), 7.44 - 7.20 (m, 2H, CH). ¹³C NMR (101 MHz, DMSO-d6): δ = 44.24, 50.19, 69.28, 114.96, 121.31, 129.98, 158.70.

**Benzyl glycidyl ether:** ¹H NMR (DMSO-d6, 400 MHz): δ = 2.56 (dd, *J =* 5.2, 2.7 Hz, 1H, CH), 2.73 (t, *J* = 4.7 Hz, 1H, CH), 3.07 - 3.21 (m, 1H, CH), 3.30 (dd, *J* = 11.5, 6.4 Hz, 1H, CH), 3.75 (dd, *J =* 11.5, 2.7 Hz, 1H, CH), 4.52 (s, 2H, CH), 7.26-7.39 (m, 5H, CH). ¹³C NMR (101 MHz, DMSO-d6): δ = 43.88, 50.76, 71.27, 72.62, 127.95, 128.00, 128.73, 138.68.

**(Benzoyloxymethyl)oxirane:** ¹H NMR (DMSO-d6, 400 MHz): δ = 2.75 (dd, *J* = 5.0, 2.6 Hz, 1H, CH), 2.86 (t, *J* = 4.6 Hz, 1H, CH), 3.34 - 3.38 (m, 1H, CH), 4.10 (dd, *J* = 12.4, 6.5 Hz, 1H, CH), 4.66 (dd, *J* = 12.3, 2.7 Hz, 1H, CH), 7.56 (t, *J* = 7.7 Hz, 2H, CH), 7.67 - 7.71 (m, 1H, CH), 7.99 - 8.01 (m, 2H, CH). ¹³C NMR (101 MHz, DMSO-d6): δ = 44.32, 49.50, 129.29, 129.70, 129.80, 133.99, 165.93.

**2-Methyl-2-(phenoxymethyl)oxirane:** ¹H NMR (DMSO-d6, 400 MHz): δ = 1.38 (s, 1H, CH), 2.70 (d, *J* = 4.9 Hz, 1H, CH), 2.80 (d, *J* = 4.9 Hz, 1H, CH), 3.86 (d, *J* = 10.8 Hz, 1H, CH), 4.13 (d, *J* = 10.8 Hz, 1H, CH), 6.92 - 6.96 (m, 3H, CH), 7.26 - 7.31 (m, 2H, CH). ¹³C NMR (101 MHz, DMSO-d6): δ = 18.67, 51.28, 55.76, 71.70, 115.04, 121.29, 129.95, 158.85.

### Example 3

### Determining Enantiomeric Ratios

To determine the ratio of R and S isomers of the epoxides formed from the oxidation of the alkenes, samples were analyzed for the presence of the epoxides and alkenes by gas chromatography and/or HPLC-MS (CHIRALCEL OD-H, 250 × 4.6 mm, 5 µm column (Daicel, Japan) was used for separation) as described above. The enantiomeric ratio of epoxides produced was determined by injecting a sample of the headspace gas onto a GC equipped with a chiral separation column (FS capillary column LIPODEX E ID: 0.25 mm, length: 25 m, Macherey Nagel, Germany) and a flame ionization detector. Method was programmed as follows: injection temperature 150 °C, split ratio 20. Flow control mode: linear velocity 35 cm/sec. Temperature program: 40 °C hold for 5 min, ramp 2 °C /min to 60 °C and hold for 3 min, ramp 2 °C /min to 80 °C and hold for 5 min, ramp 5 °C /min to 150 °C and hold until elution. Before use, the column was burned for about ~ 24 h near the max allowed temperature.

Table 3 provides information on the enantiomeric ratio of epoxides produced by PmlABCDEF (Pml), KatABCDEF (Kat) and MilABCDEF (Mil) Mediated mediated oxidation of alkenes.

**TABLE 4. Enantiomeric Ration of Epoxides Produced by PmlABCDEF (Pml), KatABCDEF (Kat) and MilABCDEF (Mil) Mediated Oxidation of Alkenes.**

| | | | |
|---|---|---|---|
| | | HPLC (CHIRALCEL OD-H) | GC (LIPODEX-E 25m × 0.25mm ID) |
| Product | enantiomer | ee | ee |
| 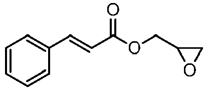 | ***S*** | 99 % | - |
| 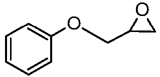 | ***S*** | 77 % | - |
| 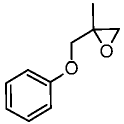 | ***S*** | - | 99 % |
| 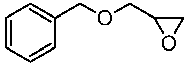 | ***S*** | 93 % | - |
| 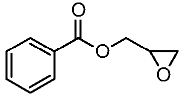 | ***S*** | 99 % | - |
| 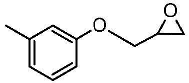 | ***S*** | 65 % | - |
| 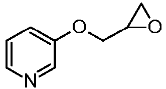 | ***S*** | 85 % | |

### List of sequences

| | | |
|---|---|---|
| SEQ ID NO:1 | >KatA | MTDTGTPFDQLPRYVRVRSEPDATFVEFDFAIGYPELFVEL VLPRQAFEQFCQHNHVRHMDAAMAQAVDDDMHKWRYGD SQ |
| SEQ ID NO:2 | >KatB | MNIEIKTTSVEPIRHTFSHTRRRFGDKPASRYQEASFDLESA RNFHYRPLWQPDKLLNDPSRTAVVMADWYRVSDPRQFYY GPYVQTRAKMQENAEHDFVFCEKRNLLAGLDPHSREQIAR LLLPLRHAEMGANMNNSGIAADGFGTSVTQMHMFHAMDRL AIAQYLSRIGLMLEDDGLALLAQAKQQWLEDPAWQGVRRY VEDSLVIKDWFELTLAQNLVSDGLLYPLLFGKFDEQLSAAGH GQVGMLTEFMRLWFGETQRWVDALVKTVAAESPANRELLE RWIATWRGRALEALAPLAAIGPGHEALQAVDNEFGARLKKL GLTGAAA |
| SEQ ID NO:3 | >KatC | MTSLVYIAFQDNDNARYIVEAITEDNPHAEVQYQPAMIRVQA EQRLEIQRATVEEKIGRTWDVQEMLIDVITLGGNVEEDEDSF TLHWN |
| SEQ ID NO:4 | >KatD | MAAKRLNQKEKYRCMTRDLGWEPSYQSKEDIFPYERFEGI KITDWDKWEDPFRLTMDAYWKYQAEKEKKLYAILDAFSQN NGHTNISDARYVNALKLFLSGVTPLEYQAYQGFARVGRHFG GAGARVACQMQAIDELRHVQTQIHAMSHYNKHFNGLHDFA HMHDRVWFLSVPKSFFEDARSAGPFEFLTAISFSFEYVLTNL LFVPFMSGAAFNGDMATVTFGFSAQSDEARHMTLGLEVIKF MLEQHEDNVPIIQRWIDKWFWRGYRLLTLVGMMMDYMLPN KVMSWAEAWKVYFEDAGGALFKDLERYGIRPPKYVEQAQI GKDHISHQAWSIFYQYSQATNFHTWMPTDEELDWLSAKYP DTFDAVYRPRYEHWRALQEKGERFYNPSLPMLCQICQIPLA FTEPDDPTTLSHRSHEHEGERYHFCSDGCCEIFQYEPMKYI QAWLPVHQILQGNCGGADVEAVVRDYYNINPGQDNFEYQD SPEHQRWQAWHPQTKAPA |
| SEQ ID NO:5 | >KatE | MPVTAIGAYSAQPLDSQQHFNGAQLVYLGWDRHLMFCAPF TFPLPPDMAFADFLEQVVKPAIAPHPDARQVNFAEARWRLD GQPFTPELQLSLIANGIAHKSLLNLVTPGLNGLAGSSN |
| SEQ ID NO:6 | >KatF | MSYLVTIEPTGEQIEVSPGQTILQAALRQGVWLPFACGHGT CATCKVQVLEGEVDIGAASTFALMDMEREEGKILACCAMPT SDLVIEADIDVDPDFQAFPVEDYRAVVSQLVDLSPTIKGVHL KLDRPMAFQAGQYINLQLPGIEGTRAFSLANPPSRADELEL |
| | | HVRLVEGGLVTGHIHQALKVGDGLELSGPYGQFFVRGSQP GGLIFIAGGSGLSSPQSMIVDLLEQGDERPMVLFQGARNRA ELYNHGLFEELALRHSNFRYVPALSQAADDEAWQGFRGYV HDAAKQHFDARFSANKAYLCGPPPMIDAAITCLMQGRLFER DIFMERFYSAADGATDGQRSALFKRI |
| SEQ ID NO:7 | >MilA | MNLPDESLDMQPADLDTRKRFVRVTGSRGSNFVEFDFAIG EPELFVEMILTHEAFDEFCRTNQVESMPALPEVDGEDDEAG QWDWRLADATQVRFKN |
| SEQ ID NO:8 | >MilB | MHIDLRTVSIQPLRQTFDHLARRFGDKPASRYQEGSYDIQA AENLHYRPTWDPDQALYDAGITKIVMSDWYALKDPRQFYYS TYTLARARQQENTEANFSFVETRGLADMLPDDLRRRALNLL VPLRHAAWGANLNNTFICSYGYGTVFTQPCMYHAMDNLGI AQYLSRIGLLLGGTEALEEGKAAWMDGDAWQGLRRYTEDC LVVRDPLELFVAQNVAMDGLLYPLVYERIVDEQFAANGGSV VAMLAQFMSDWFDETRKWVDAVMKVAAAESEQNREQLQA WTITWRDRAAAALLPVIEQAFAGQADEVMGEQLAAFKARLD KTGVVV |
| SEQ ID NO:9 | >MilC | MSTVFIALQTNEETRPIIEAIELDNPEAVVNREPAMVKINAPG RLVIRRETIEEQLGRSFDMQELNVNLITLSGNVVEDEDSLTL SWGN |
| SEQ ID NO:10 | >MilD | MDMKTANKKMNAKDKYRLLTRDLAWDPSYRTEEEIFPYIAY EGLKIHDWNKWEDPFRLTMDAYWKYQAEKERKFYAIIDAHA QNNGHLNITDARYLSALKIFLQAISPAEYSSHKGFARAGREF RGVGTQVACQMQAIDELRHAQTQIHALSNYNKFYNGFHAF ADQRDRIWYTSVARSFFDDAMSAGPFEFMIAIGFSFEYVLT NLLFVPFMSGAAYNGDMATVTFGFSAQSDEARHMTLGLECI KFMLEQDPDNLPIVQGWIDKWFWRGFRVLGLVSTMMDYML PKRVMSWREAWEIYGAENGGALFKDLARYGIRPPKSWDDA EAAIDHMSHQFMLGFYQWSFGTSFHAWIPSDEDLQWLSAK YPTTFDKYYRPRWEHIKKLEAAGTPLKNYGLAKLCQCCQLP CVFTEPDDPTLICHRETEYKGDKYHFCSDHCQSIFENEPEK YIQAWLPMPALFQAPTNGDLGAWMDWVSLKDGQDNGDFA DSADRRNFEMWRGMATSNQ |
| SEQ ID NO:11 | >MilE | MPVVARKEYVGVARDRVENFNGKQLVYVSWDHHLLFAAPF LLCLPPQTRFAELLETALKPLLQADPDAAVDWQAVQWLKG NQPWVPNFEASLADNGIGHKDQLRFKTPGLNSICAVS |
| SEQ ID NO:12 | >MilF | MSYELTIEPLGQTIEIEEGQTILDAALRAGIYLPHACCHGLCA TCKIQVVDGEIEHGEASSFALMDFEREEKKCLACCATAESD LVIEAEIDEDPDAENLPVRDFAGTVSRIETLTPTIKGVWIKLD GAGMHFQAGQYINLQLPDGIGSRAFSVASAPADGSEIELNIR IVPGGRGTAYVHEQLRVGERVSITGPYGRFFVKKSARTPVIF MGGGSGLSSPRSMILDLLAEGCELPITLIYGQRNRDELYYHE EFLAMARQHANFSYVPALSHEPEDSDWQGFRGFVHDAAKA HFDNDFRGHKAYLCGPPLMIDSCISTLMQGRLFERDIYTEKF ISAADAQQVRSPLFKAV |
| SEQ ID NO:13 | >PmIA | MSEKSLGLEPFELDTRRKFVRVTGQRGDKFIEFDFAIGEPEL FVEMILTPDAFAEFCEANQVEQMPAVTAQGEDTEDEQWDW RLADATQVRFKTE |
| SEQ ID NO:14 | >PmIB | MQIDLRTVTIEPLRVGYDHLIRRFGNKPASRYQEGSYDLQAA ENLHYRPTWDPDQELYDASFSKILMPDWYDLKDPRQFYYN PYTLTRARQQENAEANFKFVETRGLAELLPEQLREAALKLL VPLRHAAWGANQNNTLMCGYGYGAVFTQPCIYHAMDNLG TAQYLSRLGLLLGGTEALDAGKDAWMNDRAWQGLRRYTE DTLVIRDPIELFVAQNLALDGLLYPLVYERIVDDLFAAEGGTA VAMLCQFMREWFDETRKWVDAVIKTMAAESEHNREQLQN WTRHWRDRATAALLPVIEQVLADRADAVMEEQLLAFKARLD KTGLVT |
| SEQ ID NO:15 | >PmIC | MSTVFIALQANEETRPIIEAIELDNPEAVVNREPAMVKINAPG RLVIQRETIEEQMGRSYDMQELNVNLITLSGNVEEDEDSLTL TWAD |
| SEQ ID NO:16 | >PmID | MDMKTANKKLGAKEKYRLLTRDLAWEPTYRNKEEIFPYIQY EGLKIHDWSKWEDPFRLTMDAYWKYQAEKERKFYAIIDAHA QNNGHLNITDARYLSALKIFLQAISPGEYSAHKGFARVGREF AGAGTQVACQMQAIDEIRHAQTQVHALSNYNKFYNGFHAF AEQRDRIWYTSVARSFFDDAMSAGPFEFMMAIGFSFEYVLT NLLFVPFMSGAAYNGDMATVTFGFSAQSDEARHMTLGLECI KFMLEQDPDNLPIVQGWIDKWFWRGIRVLSLVSTMMDYML PKRVMSWREAWNIYGAENGGALFRDLARYGIRPPKHWDH AVDAIDHMSHQFMLALYQWKFGTAFHVWVPSDEDMDWLS AKYPDTFDKYYRPRWDYIKQQEAASGAPFNNFGLPKLCQC CQLPTLFTEPGDPTLICHRQSEYKGDVYHFCSDGCKAIFDN |
| | | EPEKYIQAWLPMPGLMQAPLHGDLGAWMDWAFLKDGQDN GDFATSQDRANFERWRAQSTSNQ |
| SEQ ID NO:17 | >PmIE | MPVVARKEYVGVARDRIENFNGKQLVFVSWDHHLLFAAPM MFCVEPQSTLGELIDNHLMPLLRIDADAAAIDWDKAEWLKG TEPWVPDFSASLEVNGIGHKVQLRFKTPQLNTVCAVR |
| SEQ ID NO:18 | >PmIF | MSYELTIEPLGQTIEIEEGQTILDAALRAGIYLPHACGHGLCA TCKVQVVDGEIEHGEASTFALMDFEREEQKCLACCATAESD IVIEAEVDEDPDSLNLPVQDFYGVVSRIESLTPTIKGVWIKLE NAGDMQFQAGQYINLMLPDEIGSRAFSIASVPGADELELNIR VVPGGRGTLYVHEQMRVGERVHLTGPYGRLFVKKSADLPV IFMAGGSGLSSPRSMILDLLNEGFARPITLIYGQRNREELYY HDEFLALGEQHGNFTYVPALSHEPEGSDWQGARGFVHEVA KNHFDNDFLEHKAYLCGPPLMIESCIGALMQGRLFERDIYTE KFISAADAQQVHSPLFKAI |

### References

1. R. H. Holm, P. Kennepohl, E. I. Solomon. Chem. Rev., 1996, 96, 2239.
2. Bruijnincx PC, van Koten G, Gebbink RJ. Chem. Soc. Rev. 2008;37(12):2716-44.
3. Lundberg M, Borowski T. Coordination chemistry reviews. 2013 Jan 1;257(1):277-89.
4. Buongiorno D, Straganz GD. Coordination chemistry reviews. 2013 Jan 15;257(2):541-63.
5. K. D. Koehntop, J. P. Emerson and L. Que, Jr, J. Biol. Inorg. Chem., 2005, 10, 87.
6. Ishikawa N, Tanaka H, Koyama F, Noguchi H, Wang CC, Hotta K, Watanabe K. Angewandte Chemie International Edition. 2014 Nov 17;53(47):12880-4.
7. An CY, Li XM, Luo H, Li CS, Wang MH, Xu GM, Wang BG. Journal of Natural Products. 2013 Oct 25;76(10):1896-901.
8. Seo MJ, Zhu D, Endo S, Ikeda H, Cane DE. Biochemistry. 2011 Mar 15;50(10):1739-54.
9. Hollenhorst MA, Bumpus SB, Matthews ML, Bollinger Jr JM, Kelleher NL, Walsh CT. Journal of the American Chemical Society. 2010 Nov 10;132(44):15773-81.
10. Wang C, Chang WC, Guo Y, Huang H, Peck SC, Pandelia ME, Lin GM, Liu HW, Krebs C, Bollinger Jr JM. Science. 2013 Nov 22;342(6161):991-5.
11. Liu G, Zhao YL, He F, Zhang P, Ouyang X, Tang H, Xu P. Nature Communications. 2021 Feb 26;12(1):1301.
12. Bassan A, Blomberg MR, Siegbahn PE. Chemistry-A European Journal. 2003 Sep 5;9(17):4055-67.
13. Han J, Kim SY, Jung J, Lim Y, Ahn JH, Kim SI, Hur HG. Applied and Environmental Microbiology. 2005 Sep;71(9):5354-61.
14. Boyd DR, Bugg TD. Organic & biomolecular chemistry. 2006;4(2):181-92.
15. Leahy JG, Batchelor PJ, Morcomb SM. FEMS microbiology reviews. 2003 Oct 1;27(4):449-79.
16. Nichol T, Murrell JC, Smith TJ. European journal of inorganic chemistry. 2015 Jul;2015(21):3419-31.
17. Osborne CD, Haritos VS. Molecular phylogenetics and evolution. 2019 Oct 1;139:106527.
18. Rosenzweig AC. Nature. 2015 Feb 19;518(7539):309-10.
19. Murray LJ, Lippard SJ. Accounts of chemical research. 2007 Jul 17;40(7):466-74.
20. Colby J, Stirling DI, Dalton HO. Biochemical Journal. 1977 Aug 1;165(2):395-402.
21. Merkx M, Kopp DA, Sazinsky MH, Blazyk JL, Muller J, Lippard SJ. Angewandte Chemie International Edition. 2001 Aug 3;40(15):2782-807.

## Claims

**1.** A method for preparing a single enantiomeric species of an epoxide comprising contacting an alkene with a monooxygenase consisting of six subunits with amino acid sequences from the group SEQ ID NO:1-6, SEQ ID NO:7-12, and SEQ ID NO:13-18, and recovering said epoxide produced.

**2.** The method for using monooxygenases according to claim 1, wherein the method comprises the steps:
a) the nucleic acid selection from the group encoding the SEQ ID NO:1-6, SEQ ID NO:7-12, and SEQ ID NO:13-18;
b) construction of a DNA vector harbouring the selected nucleic acid;
c) host cell transformation with the DNA vector from step (b);
d) preparation a single enantiomeric species of an epoxide by incubation of the host cell from step (c) with the alkene.

**3.** The method according to previous claims, wherein the alkene derivatives have the following general formula (1): 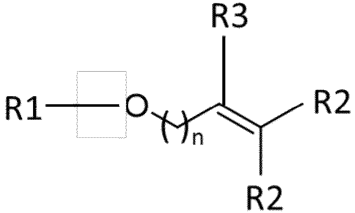 wherein R1 is C1-C12 alkyl, C1-C12 substituted alkyl, phenyl, (CH2)n-Ph, or substituted (CH2)n-Ph; wherein in said (CH2)n-Ph, n = 0, 1, 2, 3 to 10, Ph is phenyl; C1-C12 carboxyl, benzoyl or substituted benzoyl, pyridyl or substituted pyridyl, pyrimidyl or substituted pyrimidyl, thiophenyl or substituted thiophenyl,
R2 is H, CH3;
when R2 is H, R3 is H or CH3
n - 1-12.

**4.** The method according to claim 3, wherein the alkene derivatives have the following general formula (2): 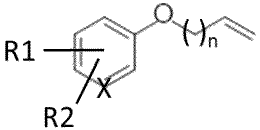 wherein R1 and R2 is H, F, Cl, Br, I, C1-C10 alkyl, C1-C10 substituted alkyl, -(CH2)n-Ph, or substituted -(CH2)n-Ph; wherein in said -(CH2)n-Ph, n = 0, 1, 2, 3 to 10, Ph is phenyl; alkoxy group with C1-C10 alkyl or C1-C10 substituted alkyl; -SCH3, -CCH3, -CN, cyclopropyl, cyclobutyl, cyclopentyl, cyclohexyl;
substituents R1 and R2 can be identical or different;
X-CorN;
n - 1-12.

**5.** The method according to claim 3, wherein the alkene derivatives have the following general formula (3): 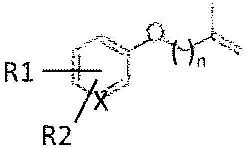 wherein R1 and R2 is H, F, Cl, Br, I, C1-C10 alkyl, C1-C10 substituted alkyl, -(CH2)n-Ph, or substituted -(CH2)n-Ph; wherein in said -(CH2)n-Ph, n = 0, 1, 2, 3 to 10, Ph is phenyl; alkoxy group with C1-C10 alkyl or C1-C10 substituted alkyl; -SCH3, -CCH3, -CN, cyclopropyl, cyclobutyl, cyclopentyl, cyclohexyl;
substituents R1 and R2 can be identical or different;
X-CorN;
n - 1-12.

**6.** The method according to claim 3, wherein the alkene derivatives have the following general formula (4): 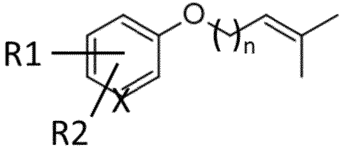 wherein R1 and R2 is H, F, Cl, Br, I, C1-C10 alkyl, C1-C10 substituted alkyl, -(CH2)n-Ph, or substituted -(CH2)n-Ph; wherein in said -(CH2)n-Ph, n = 0, 1, 2, 3 to 10, Ph is phenyl; alkoxy group with C1-C10 alkyl or C1-C10 substituted alkyl; -SCH3, -CCH3, -CN, cyclopropyl, cyclobutyl, cyclopentyl, cyclohexyl;
substituents R1 and R2 can be identical or different;
X-CorN;
n - 1-12.

**7.** The method according to claim 3, wherein the alkene derivatives have the following general formula (5): 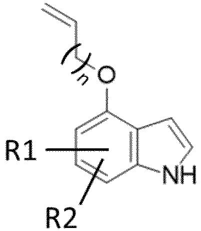 wherein R1 and R2 is H, F, Cl, Br, I, C1-C10 alkyl, C1-C10 substituted alkyl, -(CH2)n-Ph, or substituted -(CH2)n-Ph; wherein in said -(CH2)n-Ph, n = 0, 1, 2, 3 to 10, Ph is phenyl; alkoxy group with C1-C10 alkyl or C1-C10 substituted alkyl; -SCH3, -CCH3, -CN, cyclopropyl, cyclobutyl, cyclopentyl, cyclohexyl;
substituents R1 and R2 can be identical or different;
n - 1-12.

**8.** The method according to claim 2, wherein the alkene derivatives have the following general formula (6): 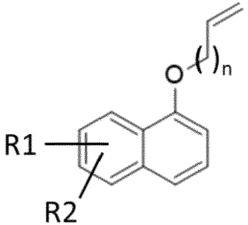 wherein R1 and R2 is H, F, Cl, Br, I, C1-C10 alkyl, C1-C10 substituted alkyl, -(CH2)n-Ph, or substituted -(CH2)n-Ph; wherein in said -(CH2)n-Ph, n = 0, 1, 2, 3 to 10, Ph is phenyl; alkoxy group with C1-C10 alkyl or C1-C10 substituted alkyl; -SCH3, -CCH3, -CN, cyclopropyl, cyclobutyl, cyclopentyl, cyclohexyl;
substituents R1 and R2 can be identical or different;
n - 1-12.

**8.** A method according to claim 3 for producing an enantiomeric excess of epoxide enantiomers comprising contacting an alkene with a monooxygenase.

**9.** The method according to claim 8 wherein said desired enantiomeric excess (ee) of the S enantiomer is more than 40%.

**10.** The method according to claim 8 wherein said desired enantiomeric excess (ee) of the S enantiomer is more than 90%.
